(19) Europäisches Patentamt
European Patent Office
Office européen des brevets

(11) EP 1 521 789 B1

(12) EUROPÄISCHE PATENTSCHRIFT

(45) Veröffentlichungstag und Bekanntmachung des Hinweises auf die Patenterteilung:
**28.01.2015 Patentblatt 2015/05**

(21) Anmeldenummer: **03762603.3**

(22) Anmeldetag: **03.07.2003**

(51) Int Cl.:
***C08G 18/02*** *(2006.01)* ***C08G 18/20*** *(2006.01)*
***C07D 229/00*** *(2006.01)*

(86) Internationale Anmeldenummer:
**PCT/EP2003/007096**

(87) Internationale Veröffentlichungsnummer:
**WO 2004/005364 (15.01.2004 Gazette 2004/03)**

(54) **URETDIONGRUPPEN AUFWEISENDE ISOCYANATE**
ISOCYANATES CONTAINING URETDION GROUPS
ISOCYANATES PRESENTANT DES GROUPES URETDIONE

(84) Benannte Vertragsstaaten:
**AT BE BG CH CY CZ DE DK EE ES FI FR GB GR HU IE IT LI LU MC NL PT RO SE SI SK TR**

(30) Priorität: **04.07.2002 DE 10230063**
**17.09.2002 DE 10243029**
**17.09.2002 DE 10243030**

(43) Veröffentlichungstag der Anmeldung:
**13.04.2005 Patentblatt 2005/15**

(73) Patentinhaber: **Bayer Intellectual Property GmbH**
**40789 Monheim (DE)**

(72) Erfinder:
- **LAAS, Hans-Josef**
**51467 Bergisch Gladbach (DE)**
- **HALPAAP, Reinhard**
**51519 Odenthal (DE)**
- **RICHTER, Frank**
**51373 Leverkusen (DE)**
- **KÖCHER, Jürgen**
**40764 Langenfeld (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**EP-A- 0 317 744** **EP-A- 0 417 603**
**EP-A- 0 639 598**

EP 1 521 789 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft neue Uretdiongruppen aufweisende Verbindungen, ein Verfahren zu ihrer Herstellung durch Dimerisierung aliphatischer und/oder cyclo-aliphatischer Mono- und/oder Diisocyanate mit ausschließlich sekundär und/oder tertiär gebundenen Isocyanatgruppen in Gegenwart von salzartigen Oligomerisierungskatalysatoren, die im Anion 1,2,3- und/oder 1,2,4-Triazolatstrukturen enthalten, sowie die Verwendung von nach diesem Verfahren aus solchen Mono- und/oder Diisocyanaten mit ausschließlich sekundär und/oder tertiär gebundenen Isocyanatgruppen erhältlichen Uretdionpolyisocyanaten als Ausgangskomponente für Polyurethankunststoffe, insbesondere als Isocyanatkomponente zur Herstellung von Uretdionpulverlackvernetzern.

Die Herstellung von Polyisocyanaten mit Uretdionstruktur durch katalytische Dimerisierung und gegebenenfalls gleichzeitige Trimerisierung monomerer aliphatischer oder cycloaliphatischer Diisocyanate ist bekannt. Ein umfassender Überblick über die technisch relevanten Dimerisierungsverfahren des Standes der Technik und die dabei zum Einsatz gelangenden Katalysatoren bzw. Katalysatorsysteme findet sich in J. Prakt. Chem. 336 (1994) 185 - 200.

Unter den lichtechten Uretdionpolyisocyanaten sind die linearen, d.h. Isocyanuratgruppen-freien Dimerisate insbesondere cycloaliphatischer Diisocyanate von hohem Interesse. Sie stellen bevorzugte Ausgangsverbindungen zur Herstellung blockierungsmittelfreier Polyurethan(PUR)-Pulverlackvernetzer dar (z.B. EP-A 45 996, EP-A 639 598 oder EP-A 669 353).

Während zur linearen katalytischen Dimerisierung aliphatischer und/oder cycloaliphatischer Diisocyanate, die mindestens eine primär gebundene Isocyanatgruppe aufweisen, wie z. B. 1,6-Diisocyanatohexan (HDI) oder 1-Isocyanato-3,3,5-trimethyl-5-isocyanatomethylcyclohexan (Isophorondiisocyanat; IPDI), verschiedene Verfahren existieren (z.B. EP-A 45 995, EP-A 317 744, EP-A 735 027 und EP-A 896 973), die zum Teil auch im technischen Maßstab bei der Herstellung von Pulverlackvemetzern

Anwendung finden, sind isocyanuratgruppenfreie Uretdionpolyisocyanate aus aliphatischen und/oder cycloaliphatischen Diisocyanaten mit ausschließlich sekundär und/oder tertiär gebundenen Isocyanatgruppen bislang nicht bekannt. Die üblichen Dimerisierungskatalysatoren zeigen gegenüber solchen Diisocyanaten keine oder eine derart geringe Aktivität, dass sich mit ihrer Hilfe auch unter Einsatz sehr hoher Katalysatorkonzentrationen die entsprechenden Dimerisate nicht oder lediglich in verschwindend geringer Ausbeute herstellen lassen.

Obwohl in einigen der genannten Veröffentlichungen zur katalytischen Dimerisierung von Isocyanaten, z. B. in EP-A 178 520, DE-A 34 20 114, EP-A 317 744 oder EP-A 896 973, auch Diisocyanate wie 2,4'- bzw. 4,4'-Diisocyanatodicyclohexylmethan, 1,3- bzw. 1,4-Diisocyanatocyclohexan, 1,3-Diisocyanatocyclobutan oder 1,3-Diisocyanato-2(4)-methylcyclohexan als mögliche Ausgangsverbindungen genannt sind, wurden Uretdionpolyisocyanate aus cycloaliphatischen Diisocyanaten, die keine primär gebundenen Isocyanatgruppen aufweisen, aufgrund der fehlenden Aktivität der bisher bekannten Dimerisierungskatalysatoren noch nie konkret beschrieben.

Aufgabe der vorliegenden Erfindung war es daher, ein neues Verfahren zur Herstellung von Uretdionpolyisocyanaten aus aliphatischen und/oder cycloaliphatischen Mono- und/oder Diisocyanaten mit ausschließlich sekundär und/oder tertiär gebundenen Isocyanatgruppen zur Verfügung zu stellen, das unter Verwendung hochreaktiver und selektiver Katalysatoren möglichst lineare, vorzugsweise isocyanuratgruppenfreie Produkte liefert, wie sie als Ausgangskomponenten für Uretdionpulverlackvernetzer benötigt werden.

Gegenstand der vorliegenden Erfindung sind durch Dimerisierung aliphatischer und/oder cycloaliphatischer Mono- und/oder Diisocyanate mit ausschließlich sekundär und/oder tertiär gebundenen Isocyanatgruppen erhältliche Uretdiongruppen aufweisende Verbindungen mit einem molaren Anteil an Isocyanuratstrukturen, bezogen auf die Summe an Uretdion- und Isocyanuratgruppen, von maximal 10 %.

Gegenstand der vorliegenden Erfindung ist auch ein Verfahren zur Dimerisierung von Verbindungen mit ausschließlich sekundär und/oder tertiär gebundenen Isocyanatgruppen, bei dem aliphatische und/oder cycloaliphatische Mono- und/oder Diisocyanate, als Ausgangsverbindungen, mit ausschließlich an sekundären und/oder tertiären C-Atomen gebundenen NCO-Gruppen in Gegenwart mindestens eines salzartigen Oligomerisierungskatalysators, der im Anion 1,2,3- und/oder 1,2,4- Triazolatstrukturen enthält, umgesetzt werden.

Gegenstand der Erfindung ist auch die Verwendung von nach diesem Verfahren aus solchen Mono- und/oder Diisocyanaten mit ausschließlich sekundär und/oder tertiär gebundenen Isocyanatgruppen erhältlichen Uretdionpolyisocyanaten als Ausgangskomponenten für Polyurethankunststoffe, insbesondere als Isocyanatkomponenten zur Herstellung von Uretdionpulverlackvernetzern.

Ausgangsverbindungen für das erfindungsgemäße Verfahren sind beliebige aliphatische und/oder cycloaliphatische Mono- und Diisocyanate mit ausschließlich sekundär und/oder tertiär gebundenen Isocyanatgruppen, die nach beliebigen Verfahren, z. B. durch Phosgenierung oder auf phosgenfreiem Weg, beispielsweise durch Urethanspaltung, hergestellt werden können. Die Bezeichnung aliphatisch bzw. cycloaliphatisch bezieht sich dabei lediglich auf die Art der Isocyanatgruppentragenden Kohlenstoffatome, d.h. im Molekül können durchaus auch aromatische Strukturen vorhanden sein. Geeignete Ausgangsisocyanate sind beispielsweise Monoisocyanate, wie 2-Isocyanatopropan, 2-Isocyanato-2-methylpropan, Isocyanatocyclohexan, 1-Isocyanato-4-isopropenyl-1-methylcyclohexan, 4-(1-Isocyanato-1-methylethyl)-1-methyl-1-cyclohexen, 1,3-Dimethyl-5-isocyanatoadamantan oder 1-(1-Isocyanato-1-methylethyl)-3-isopropenylbenzol (TMI), oder Diisocyanate wie 1,3-bzw. 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1,8-Diisocyanato-p-menthan, 4,4'-Diisocyanato-1,1'-dicyclohexyl-, 4,4'-Diisocyanato-3,3'-dimethyl-1,1'-dicyclohexyl-, 4,4'-Diisocyanato-2,2',5,5'-tetramethyl-1,1'-dicyclohexyl-, 4,4-Diisocyanatodicyclohexylmethan, 4,4'-Diisocyanato-3,3'-dimethyldicyclohexylmethan, 4,4'-Diisocyanato-3,3',5,5'-tetramethyldicyclohexyl-methan, 1,3-Diisocyanatoadamantan, 1,3-Dimethyl-5,7-diiocyanatoadamantan, 1,3-und 1,4-Bis(1-isocyanato-1-methylethyl)-benzol (TMXDI), Bis(4-(1-isocyanato-1-methylethyl)phenyl)-carbonat sowie beliebige Gemische solcher Mono- und Diisocyanate. Weitere ebenfalls geeignete Ausgangsisocyanate mit ausschließlich sekundär und/oder tertiär gebundenen Isocyanatgruppen finden sich darüberhinaus beispielsweise in Justus Liebigs Annalen der Chemie Band 562 (1949) S. 75 -136.

Bevorzugte Ausgangsisocyanate für das erfindungsgemäße Verfahren sind Diisocyanate der vorstehend explizit genannten Art. Besonders bevorzugte Ausgangsverbindungen sind 4,4'-Diisocyanatodicyclohexylmethan, 1,3-bzw. 1,4-Diisocyanatocyclohexan oder TMXDI. Ganz besonders bevorzugtes Ausgangsisocyanat ist 4,4'-Diisocyanatodicyclohexylmethan.

Als Oligomerisierungskatalysatoren kommen beim erfindungsgemäßen Verfahren beliebige salzartige Verbindungen zum Einsatz, die im Anion 1,2,3- und/oder 1,2,4-Triazolatstrukturen enthalten. Hierbei handelt es sich um Verbindungen, die im Anion Triazolatstrukturen der allgemeinen Formeln (I) und/oder (II)

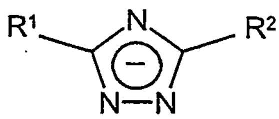

(I)

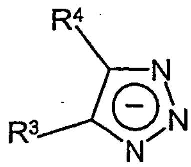

(II)

enthalten, in welchen

$R^1$, $R^2$, $R^3$ und $R^4$ für gleiche oder verschiedenen Reste stehen und jeweils ein Wasserstoffatom, ein Halogenatom aus der Reihe Fluor, Chlor oder Brom oder eine Nitrogruppe, einen gesättigten oder ungesättigten aliphatischen oder cycloaliphatischen, einen gegebenenfalls substituierten aromatischen oder araliphatischen Rest, der bis zu 20 Kohlenstoffatome und gegebenenfalls bis zu 3 Heteroatome aus der Gruppe Sauerstoff, Schwefel, Stickstoff enthalten und gegebenenfalls durch Halogenatome oder Nitrogruppen substituiert sein kann, bedeuten,

und wobei

$R^3$ und $R^4$ in Formel (II) auch gemeinsam mit den Kohlenstoffatomen des 1,2,3-Triazolatfünfringes anellierte Ringe mit 3 bis 6 Kohlenstoffatomen bilden können.

Bevorzugte Oligomerisierungskatalysatoren sind solche, die im Anion Triazolatstrukturen der allgemeinen Formel (I) enthalten, in welcher

$R^1$ und $R^2$ für gleiche oder verschiedenen Reste stehen und jeweils ein Wasserstoffatom, ein Halogenatom aus der Reihe Fluor, Chlor oder Brom oder eine Nitrogruppe, einen gesättigten aliphatischen oder cycloaliphatischen, einen gegebenenfalls substituierten aromatischen oder araliphatischen Rest, der bis zu 12 Kohlenstoffatome und gegebenenfalls bis zu 3 Heteroatome aus der Gruppe Sauerstoff, Schwefel, Stickstoff enthalten und gegebenenfalls durch Halogenatome oder Nitrogruppen substituiert sein kann, bedeuten.

Als Oligomerisierungskatalysatoren ebenfalls bevorzugt sind solche, die im Anion Triazolatstrukturen der allgemeinen Formel (II) enthalten, in welcher

$R^3$ und $R^4$ für gleiche oder verschiedenen Reste stehen und jeweils ein Wasserstoffatom, ein Halogenatom aus der Reihe Fluor, Chlor oder Brom oder eine Nitrogruppe, einen gesättigten oder ungesättigten aliphatischen oder cycloaliphatischen, einen gegebenenfalls substituierten aromatischen oder araliphatischen Rest, der bis zu 12 Kohlenstoffatome und gegebenenfalls bis zu 3 Heteroatome aus der Gruppe Sauerstoff, Schwefel,

Stickstoff enthalten und gegebenenfalls durch Halogenatome oder Nitrogruppen substituiert sein kann, bedeuten, und auch gemeinsam mit den Kohlenstoffatomen des 1,2,3-Triazolatfünfringes anellierte Ringe mit 3 bis 6 Kohlenstoffatomen bilden können.

Besonders bevorzugte Oligomerisierungskatalysatoren für das erfindungsgemäße Verfahren sind Salze des 1,2,4-Triazols, des 1,2,3-Triazols und/oder des 1,2,3-Benztriazols.

Als Gegenionen zu den katalytisch aktiven. Triazolat-Anionen können die erfindungsgemäß einzusetzenden Katalysatoren beliebige Kationen enthalten. Beispielhaft seien hier genannt Alkalikationen wie $Li^+$, $Na^+$ und $K^+$, Erdalkalikationen wie $Mg^{2+}$ und $Ca^{2+}$ sowie Ammonium- oder Phosphoniumkationen, der allgemeinen Formel (III),

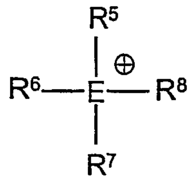

(III)

in welcher

E für Stickstoff oder Phosphor steht,

$R^5$, $R^6$, $R^7$ und $R^8$ für gleiche oder verschiedenen Reste stehen und jeweils ein Wasserstoffatom, einen gesättigten oder ungesättigten aliphatischen oder cycloaliphatischen, einen gegebenenfalls substituierten aromatischen oder araliphatischen Rest, der bis zu 24 Kohlenstoffatome und gegebenenfalls bis zu 3 Heteroatome aus der Gruppe Sauerstoff, Schwefel, Stickstoff enthalten und gegebenenfalls durch Halogenatome oder Hydroxygruppen substituiert sein kann, bedeuten, und wobei

$R^8$ auch für einen Rest der Formel (IV)

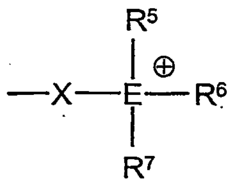

(IV)

stehen kann, in welchem

X einen zweibindigen, gegebenenfalls substituierten aliphatischen, cycloaliphatischen, araliphatischen oder aromatischen Rest mit bis zu 12 Kohlenstoffatomen bedeutet und

$R^5$, $R^6$, $R^7$ und E die oben angegebene Bedeutung haben.

Bevorzugte Kationen sind Alkaliionen oder einwertige Ammonium- oder Phosphoniumkationen, der allgemeinen Formel (III), in welcher

E für Stickstoff oder Phosphor steht und

$R^5$, $R^6$, $R^7$ und $R^8$ für gleiche oder verschiedenen Reste stehen und jeweils einen gesättigten aliphatischen oder cycloaliphatischen, einen gegebenenfalls substituierten aromatischen oder araliphatischen Rest mit bis zu 18 Kohlenstoffatome bedeuten.

Die beim erfindungsgemäßen Verfahren als Oligomerisierungskatalysatoren eingesetzten salzartigen Verbindungen sind zum Teil, z.B. in Form ihrer Natriumsalze, kommerziell erhältlich, ansonsten nach gängigen Labormethoden leicht zugänglich.

Diese Katalysatoren kommen beim erfindungsgemäßen Verfahren im Allgemeinen in Mengen von 0,01 bis 3 Gew.-%, vorzugsweise 0,1 bis 2 Gew.-%, bezogen auf die Menge an eingesetztem molekularem Isocyanat, zum Einsatz. Die Zugabe zum Reaktionsgemisch kann in Substanz erfolgen; gegebenenfalls können die Katalysatoren jedoch auch

in einem geeigneten organischen Lösungsmittel gelöst eingesetzt werden. Der Verdünnungsgrad der Katalysatorlösungen kann dabei innerhalb eines sehr breiten Bereichs frei gewählt werden. Katalytisch wirksam sind Lösungen ab einer Konzentration von 0,01 Gew.-%.

Geeignete Katalysatorlösungsmittel sind beispielsweise gegenüber Isocyanatgruppen inerte Lösungsmittel wie z.B. Hexan, Toluol, Xylol, Chlorbenzol, Essigsäureethylester, Essigsäurebutylester, Diethylenglykoldimethylether, Dipropylenglykoldimethylether, Ethylenglykolmonomethyl- oder -ethyletheracetat, Diethylenglykolethyl- und -butyletheracetat, Propylenglykolmonomethyletheracetat, 1-Methoxy-propyl-2-acetat, 3-Methoxy-n-butylacetat, Propylenglykoldiacetat, Aceton, Methylethylketon, Methylisobutylketon, Cyclohexanon, Lactone, wie ß-Propiolacton, $\gamma$-Butyrolacton, $\varepsilon$-Caprolacton und $\varepsilon$-Methylcaprolacton, aber auch Lösungsmittel wie N-Methylpyrrolidon und N-Methylcaprolactam, 1,2-Propylencarbonat, Methylenchlorid, Dimethylsulfoxid, Triethylphosphat oder beliebige Gemische derartiger Lösungsmittel.

Falls überhaupt Katalysatorlösungsmittel beim erfindungsgemäßen Verfahren eingesetzt werden, sind dies bevorzugt solche, die gegenüber Isocyanaten reaktive Gruppen tragen und im Reaktionsprodukt eingebaut werden. Beispiele für solche Lösungsmittel sind ein- oder mehrwertige einfache Alkohole, wie z. B. Methanol, Ethanol, n-Propanol, Isopropanol, n-Butanol, n-Hexanol, 2-Ethyl-1-hexanol, Ethylenglykol, Propylenglykol, die isomeren Butandiole, 2-Ethyl-1,3-hexandiol oder Glycerin; Etheralkohole, wie z. B. 1-Methoxy-2-propanol, 3-Ethyl-3-hydroxymethyl-oxetan, Tetrahydrofurfurylalkohol, Ethylenglykolmonomethylether, Ethylenglykolmonoethylether, Ethylenglykolmonobutylether, Diethylenglykolmono-methylether, Diethylenglykolmonoethylether, Diethylenglykolmonobutylether, Diethylenglykol, Dipropylenglykol oder auch flüssige höhermolekulare Polyethylenglykole, Polypropylenglykole, gemischte Polyethylen/polypropylenglykole sowie deren Monoalkylether; Esteralkohole, wie z. B. Ethylenglykolmonoacetat, Propylenglykolmonolaurat, Glycerinmono- und diacetat, Glycerinmonobutyrat oder 2,2,4-Trimethyl-1,3-pentandiol-monoisobutyrat; ungesättigte Alkohole wie z. B. Allylalkohol, 1,1-Dimethyl-allylalkohol oder Oleinalkohol; araliphatische Alkohole wie z. B. Benzylalkohol; N-monosubstituierte Amide, wie z. B. N-Methylformamid, N-Methylacetamid, Cyanacetamid oder 2-Pyrrolidinon oder beliebige Gemische derartiger Lösungsmittel.

Gegebenenfalls, insbesondere im Fall der Umsetzung von Diisocyanaten, wird die Oligomerisierungsreaktion beim erfindungsgemäßen Verfahren beim gewünschten Umsatzgrad, beispielsweise wenn 10 bis 60 % der ursprünglich im Ausgangsgemisch vorliegenden Isocyanatgruppen abreagiert haben, mit Hilfe geeigneter Katalysatorgifte abgebrochen. Solche Katalysatorgifte sind beispielsweise anorganische Säuren wie Salzsäure, phosphorige Säure oder Phosphorsäure, Säurechloride wie Acetylchlorid, Benzoylchlorid oder Isophthaloyldichlorid, Sulfonsäuren und Sulfonsäureester, wie Methansulfonsäure, p-Toluolsulfonsäure, Trifluormethansulfonsäure, Perfluorbutansulfonsäure, Dodecylbenzolsulfonsäure, p-Toluolsulfonsäuremethylester und p-Toluolsulfonsäureethylester, Mono- und Dialkylphosphate wie Monotridecylphosphat, Dibutylphosphat und Dioctylphosphat, aber auch silylierte Säuren, wie Methan-sulfonsäuretrimethylsilylester, Trifluormethansulfonsäuretrimethylsilylester, Phosphorsäure-tris-(trimethylsilylester) und Phosphorsäurediethylester-trimethylsilylester.

Die zur Abstoppung der Reaktion benötigte Menge des Katalysatorgiftes richtet sich dabei nach der molaren Menge des verwendeten Katalysators; im Allgemeinen wird eine äquivalente molare Menge des Abstoppers, bezogen auf den zu Beginn eingesetzten Oligomerisierungskatalysator, eingesetzt. Berücksichtigt man allerdings eventuell während der Reaktion auftretende Katalysatorverluste, so können zur Reaktionsstoppung auch schon 20 bis 80 mol-% des Katalysatorgiftes, bezogen auf die ursprünglich eingesetzte molare Katalysatormenge, ausreichen.

Die genannten Katalysatorgifte können sowohl in Substanz als auch in einem geeigneten organischen Lösungsmittel gelöst eingesetzt werden. Geeignete Lösungsmittel sind beispielsweise die bereits oben als mögliche Katalysatorlösungsmittel beschriebenen Lösungsmittel oder deren Gemische. Der Verdünnungsgrad kann innerhalb eines sehr breiten Bereichs frei gewählt werden, geeignet sind beispielsweise Lösungen ab einer Konzentration von 10 Gew.-%.

Neben den genannten organischen Lösungsmitteln können beim erfindungsgemäßen Verfahren auch die obengenannten Ausgangsisocyanate mit ausschließlich sekundär und/oder tertiär gebundenen Isocyanatgruppen als Lösungsmittel für die Katalysatorgifte dienen, sofern diese ausreichend inert gegenüber Isocyanatgruppen sind, so dass sich lagerstabile Lösungen herstellen lassen.

Beim erfindungsgemäßen Verfahren können gegebenenfalls auch in der Polyurethanchemie übliche Additive als Stabilisatoren mitverwendet werden. Hierbei handelt es sich beispielsweise um phenolische Antioxidantien, wie z. B. 2,6-Di-tert.-butyl-4-methylphenol, 2,4,6-Tri-tert.-butylphenol und 3,5-Di-tert.-butyl-4-hydroxyanisol, oder mit Alkyl- und/oder Arylresten trisubstituierte Phosphitstabilisatoren, wie z.B. Triphenylphosphit, Tris(nonylphenyl)phosphit, Diphenyl-isooctylphosphit, Diphenylisodecylphosphit, Diisodecyl-phenylphosphit, Diisooctyl-octylphenylphosphit, Phenylneopentylglykolphosphit, 2,4,6-Tri-tert.-butylphenyl-(2-butyl-2-ethyl-1,3-pro-pandiol)phosphit, Triisodecylphosphit, Trilaurylphosphit, Tris(tridecyl)phosphit, Diisodecyl-pentaerythritdiphosphit, Distearyl-pentaerythritdiphosphit, Bis(2,4-di-tert.-butyl-phenyl)-pentaerythritdiphosphit und Tetraphenyl-dipropylenglykoldiphosphit oder beliebige Gemische solcher Additive.

Falls diese Additive überhaupt eingesetzt werden, werden sie dem Reaktionsgemisch in einer Menge von bis zu 5 Gew.-%, vorzugsweise bis zu 3 Gew.-%, bezogen auf die Menge an eingesetzten Ausgangsisocyanaten, zugesetzt.

In einer besonderen Ausführungsform des erfindungsgemäßen Verfahrens dienen bei Raumtemperatur flüssige Additive der genannten Art, vorzugsweise die genannten flüssigen Phosphitstabilisatoren als Lösungsmittel für die eingesetzten Katalysatoren und/oder Katalysatorgifte.

Das erfindungsgemäße Verfahren wird abgesehen von gegebenenfalls mitverwendeten Katalysator- und/oder Abstopperlösungsmitteln vorzugsweise in Substanz durchgeführt. Es kann aber auch, wenn gewünscht, in Gegenwart weiterer Mengen von gegenüber Isocyanatgruppen inerten Lösungsmitteln durchgeführt werden. Geeignet sind beispielsweise die bereits oben als mögliche Katalysatorlösungsmittel beschriebenen nichtreaktiven Lösungsmittel oder beliebige Gemische dieser Lösungsmittel, die gegebenenfalls in einer Menge von bis zu 80 Gew.-%, bezogen auf die Gesamtmenge an Ausgangsisocyanaten und zugesetztem Lösungsmittel, mitverwendet werden können.

Zur Durchführung des erfindungsgemäßen Verfahrens werden die genannten Ausgangsverbindungen mit ausschließlich sekundär und/oder tertiär gebundenen Isocyanatgruppen gegebenenfalls unter Inertgas wie beispielsweise Stickstoff, gegebenenfalls in Gegenwart eines geeigneten Lösungsmittels und gegebenenfalls eines Stabilisators der genannten Art bei einer Temperatur von 0 bis 100°C, vorzugsweise 20 bis 60°C vorgelegt. Dann wird ein Oligomerisierungskatalysator bzw. eine Lösung eines Oligomerisierungskatalysators der oben genannten Art in der oben angegebenen Menge zugegeben und die Reaktionstemperatur gegebenenfalls durch eine geeignete Maßnahme (Heizen oder Kühlen) auf eine Temperatur von 20 bis 100°C, vorzugsweise von 25 bis 80°C eingestellt. Die Zugabe des Katalysators kann dabei in einer oder mehreren Portionen aber auch kontinuierlich, z. B. mit Hilfe einer geeigneten Dosierpumpe, über die gesamte Reaktionszeit erfolgen. Die Umsetzung kann gegebenenfalls bei einem angestrebten Oligomerisierungsgrad, beispielsweise bei Erreichen eines Oligomerisierungsgrades von 10 bis 60 %, vorzugsweise 10 bis 40 % durch Zugabe eines Katalysatorgiftes der beispielhaft genannten Art und gegebenenfalls nachfolgendes kurzzeitiges Erhitzen der Reaktionsmischung beispielsweise auf eine oberhalb 80°C liegende Temperatur beendet werden. Unter "Oligomerisierungsgrad" ist dabei der Prozentsatz der in der Ausgangsmischung ursprünglich vorhandenen Isocyanatgruppen (diese entsprechen 100%) zu verstehen, der während der erfindungsgemäßen Umsetzung (insbesondere durch Dimerisierung, daneben unter Trimerisierung und im Falle der Mitverwendung der beschriebenen, beispielsweise alkoholischen Katalysatorlösungsmittel durch Reaktion mit Isocyanatgruppen beispielsweise unter Urethanisierung) verbraucht wird. Der genannte Oligomerisierungsgrad wird im Allgemeinen nach einer Reaktionszeit von 30 Minuten bis 8 Stunden, vorzugsweise 1 bis 6 Stunden erreicht.

Vorzugsweise wird das Reaktionsgemisch anschließend durch Dünnschichtdestillation bei Drücken von 0,001 bis 20 mbar, bevorzugt 0,01 bis 5 mbar, unter möglichst schonenden Bedingungen, beispielsweise bei einer Temperatur von 120 bis 220°C, vorzugsweise von 140 bis 190°C, von flüchtigen Bestandteilen (überschüssigen monomeren Ausgangsisocyanaten und gegebenenfalls mitverwendeten nichtreaktiven Lösungsmitteln und Stabilisatoren) befreit.

In einer anderen, jedoch weniger bevorzugten Ausführungsform des erfindungsgemäßen Verfahrens werden die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Oligomerisierungsprodukt abgetrennt.

Auf diese Weise erhält man in Abhängigkeit von der Art der gewählten Ausgangsisocyanate farbhelle oder nahezu farblose Uretdiongruppen aufweisende Produkte, deren Gehalt an Isocyanatgruppen in Abhängigkeit vom Oligomerisierungsgrad bis zu 25,4 Gew.-%, vorzugsweise bis zu 23,9 Gew.-%, bei ausschließlicher Verwendung von Diisocyanaten als Ausgangsverbindungen von 11,2 bis 25,4 Gew.-%, vorzugsweise von 12,8 bis 23,9 Gew.-%, beträgt und die weniger als 5 Gew.-%, vorzugsweise weniger als 2 Gew.-%, besonders bevorzugt weniger als 1 Gew.-% an monomeren Ausgangsisocyanaten enthalten. Der molare Anteil an Isocyanuratstrukturen in den erfindungsgemäßen Verfahrensprodukten beträgt bezogen auf die Summe an Uretdion- und Isocyanuratgruppen vorzugsweise maximal 10 %, besonders bevorzugt maximal 8 % und ganz besonders bevorzugt maximal 5 %.

Die anfallenden Destillate, die neben nicht umgesetzten monomeren Ausgangsisocyanaten gegebenenfalls mitverwendete Lösungsmittel und Stabilisatoren sowie bei Verzicht auf den Einsatz eines Katalysatorgiftes gegebenenfalls aktiven Katalysator enthalten, können problemlos zur erneuten Oligomerisierung verwendet werden.

Gegebenenfalls kann beim erfindungsgemäßen Verfahren nach anteiliger katalytischer Oligomerisierung und Abbruch der Reaktion beim angestrebten Oligomerisierungsgrad durch Zugabe eines Katalysatorgiftes auch auf die Abtrennung des überschüssigen, nicht umgesetzten Ausgangsdiisocyanates verzichtet werden. In diesem Fall erhält man als Verfahrensprodukte farbhelle Lösungen von Uretdiongruppen enthaltenden Verbindungen in bis zu 70 Gew.-% monomerem Ausgangsisocyanat.

Das erfindungsgemäße Verfahren gestattet auf einfache Weise unter Verwendung sehr geringer Katalysatorkonzentrationen innerhalb sehr kurzer Reaktionszeiten erstmals die Dimerisierung sekundär und/oder tertiär gebundener Isocyanatgruppen.

Die nach diesem Verfahren aus Mono- und/oder Diisocyanaten mit ausschließlich sekundär und/oder tertiär gebundenen Isocyanatgruppen erhältlichen Uretdionpolyisocyanate bzw. deren Lösungen in monomeren Ausgangsdiisocyanaten stellen wertvolle Ausgangsmaterialien zur Herstellung von Polyurethankunststoffen nach dem Polyadditionsverfahren, bevorzugt zur Herstellung von Ein- oder Zweikomponenten-Polyurethanlacken, dar. Sie können dabei

auch in mit aus der Polyurethanchemie an sich bekannten Blockierungsmitteln blockierter Form als Vernetzerkomponenten für Einkomponenten-Einbrennlacke eingesetzt werden. Geeignete Blockierungsmittel sind beispielsweise die aus der Polyurethanchemie als Blockierungsmittel für Isocyanatgruppen bekannten Oxime, wie z. B. Acetonoxim, Butanonoxim und Cyclohexanonoxim, Lactame, wie ε-Caprolactam, C-H-acide Verbindungen, wie Malonsäurediethylester und Acetessigester, N-Heterocyclen, wie 1,2,4-Triazol, Dimethyl-1,2,4-tri-azol, 3,5-Dimethylpyrazol und Imidazol sowie beliebige Gemische dieser Blockierungsmittel.

Die nach dem erfindungsgemäßen Verfahren aus Mono- und/oder Diisocyanaten mit ausschließlich sekundär und/oder tertiär gebundenen Isocyanatgruppen erhältlichen Uretdionpolyisocyanate eignen sich insbesondere als Ausgangskomponenten zur Herstellung von Uretdionpulverlackvernetzern.

**Beispiele**

Die folgenden Beispiele dienen zur weiteren Erläuterung der Erfindung. Der Begriff "Oligomerisierungsgrad" bezeichnet den Prozentsatz der in der Ausgangsmischung ursprünglich vorhandenen Isocyanatgruppen (diese entsprechen 100 %), der während der erfindungsgemäßen Umsetzung, beispielsweise durch Dimerisierung und Trimerisierung, verbraucht wird.

Herstellung der Katalysatoren

**Katalysator 1:** Natrium-1,2,4-triazolat

In einer Dreihalskolben-Rührapparatur mit mechanischem Rührer, Innenthermometer und Rückflusskühler wurden 200 ml trockenes Methanol und 45 ml einer 30 gew.-%igen methanolischen Lösung von Natrium-Methanolat, entsprechend 0,25 mol Natrium-Methanolat, unter trockenem Stickstoff vorgelegt. Hierzu gab man bei Raumtemperatur portionsweise 17,4 g (0,25 mol) 1,2,4-Triazol. Nach beendeter Zugabe des 1,2,4-Triazols wurde die Reaktionsmischung 4 h lang bei Rückflußtemperatur gerührt. Anschließend wurde das Lösungsmittel bei vermindertem Druck abdestilliert und der verbleibende ölige Rückstand bei Raumtemperatur mit 200 ml Methylenchlorid versetzt. Man rührte 15 min bei Raumtemperatur und filtrierte das als Feststoff ausgefallene Produkt ab. Man erhielt 22,5 g Natrium-1,2,4-triazolat (Ausbeute: 98 % d.Th.) in Form eines farblosen Pulvers. Das Produkt war laut $^1$H-NMR-Spektrum rein und frei von eingesetztem 1,2,4-Triazol.

**Katalysator 2**: Natrium-1,2,3-triazolat

17,4 g (0,25 mol) 1,2,3-Triazol wurden nach dem für Katalysator 1 beschriebenen Verfahren in 200 ml Methanol mit einer äquivalenten Menge methanolischer Natrium-Methanolat-Lösung umgesetzt. Das Reaktionsgemisch wurde wie oben beschrieben aufgearbeitet und man erhielt 22,4 g Natrium-1,2,3-triazolat (Ausbeute: 98 % d.Th.) in Form eines fast farblosen Pulvers. Das Produkt war laut $^1$H-NMR-Spektrum rein und frei von Edukt.

**Katalysator 3:** Natrium-Benztriazolat

29,8 g (0,25 mol) Benztriazol wurden nach dem für Katalysator 1 beschriebenen Verfahren in 200 ml Methanol mit einer äquivalenten Menge methanolischer Natrium-Methanolat-Lösung umgesetzt. Das Reaktionsgemisch wurde wie oben beschrieben aufgearbeitet und man erhielt 34,2 g Natrium-Benztriazolat (Ausbeute: 97 % d.Th.) in Form eines fast farblosen Pulvers. Das Produkt war laut $^1$H-NMR-Spektrum rein und frei von Edukt.

**Katalysator 4:** Tetrabutylphosphonium-1,2,4-triazolat

In einer Dreihalskolben-Rührapparatur mit mechanischem Rührer, Innenthermometer und Rückflusskühler wurden bei Raumtemperatur unter trockenem Stickstoff 18,0 g einer 30 gew.-%igen methanolischen Natriummethanolat-Lösung, entsprechend 0,1 mol Natrium-Methanolat, vorgelegt. Man tropfte innerhalb von 20 min eine Lösung von 6,9 g (0,1 mol) 1,2,4-Triazol in 20 ml Methanol zu, rührte das Reaktionsgemisch eine Stunde nach und gab anschließend innerhalb von 20 min 41,3 g (0,1 mol) einer 71,4 gew.-%igen Lösung von Tetrabutylphosphoniumchlorid in Isopropanol (Cyphos® 443P, Fa. Cytec Industries, Neuss) zu. Unmittelbar nach Beginn der Phosphoniumsalz-Zugabe setzte die Ausscheidung von Natriumchlorid ein. Man rührte die Reaktionsmischung eine weitere Stunde bei Zimmertemperatur nach, filtrierte und engte schließlich am Rotationsverdampfer bei einer Badtemperatur von 40°C und einem Druck von ca. 1 mbar bis auf eine Menge von ca. 50 ml ein. Der Rückstand wurde erneut filtriert, und man erhielt 42,5 g einer klaren, nahezu farblosen Lösung von Tetrabutylphosphonium-1,2,4-triazolat in einem Methanol/Isopropanol-Gemisch. Der Gehalt an aktivem Katalysator betrug nach acidimetrischer Titration mit 0,1 n HCl gegen Phenolphthalein 73,0 Gew.-

%, das gaschromatografisch (GC) bestimmte Verhältnis von Methanol zu Isopropanol betrug 25,4 : 74,6 % (Flächen-%).

**Katalysator 5:** Methyltrioctylammonium-1,2,4-triazolat

Nach dem für Katalysator 4 beschriebenen Verfahren wurden 6,9 g (0,1 mol) 1,2,4-Triäzol gelöst in 20 g Methanol zuerst mit 18,0 g (0,1 mol) 30 gew.-%iger methanolischer Natriummethanolat-Lösung und anschließend mit 80,6 g einer 50 %igen Lösung von Methyltrioctylammoniumchlorid (Aliquat® 336, Cognis Deutschland GmbH&Co. KG, Düsseldorf) in Methanol, entsprechend 0,1 mol Methyltrioctylammoniumchlorid, umgesetzt. Nach Filtration, Entfernen des Lösungsmittels am Rotationsverdampfer und erneuter Filtration erhielt man 40,3 g Methyltrioctylammonium-1,2,4-triazolat als klare, hellgelbe Flüssigkeit. Der Gehalt an aktivem Katalysator betrug nach acidimetrischer Titration mit 0,1 n HCl 92,3 Gew.-%.

**Katalysator 6**: Trihexyltetradecylphosphonium-1,2,4-triazolat

In einer Dreihalskolben-Rührapparatur mit mechanischem Rührer, Innenthermometer und Rückflusskühler wurden bei Raumtemperatur unter trockenem Stickstoff 180 g einer 30 gew.-%igen methanolischen Natrium-Methanolat-Lösung, entsprechend 1,0 mol Natrium-Methanolat, vorgelegt. Man tropfte innerhalb von 45 min eine Lösung von 69 g (1,0 mol) 1,2,4-Triazol in 200 ml Methanol zu und rührte das Reaktionsgemisch 12 Stunden nach. Anschließend tropfte man innerhalb von 1 Stunde eine Lösung von 518 g (1,0 mol) Trihexyltetradecylphosphoniumchlorid (Cyphos® 3653, Fa. Cytec Industries, Neuss) gelöst in 60 g Methanol zu. Unmittelbar nach Beginn der Phosphoniumsalz-Zugabe setzte die Ausscheidung von Natriumchlorid ein. Man rührte die Reaktionsmischung über Nacht bei Raumtemperatur nach, filtrierte das ausgefallene Natriumchlorid ab und destillierte anschließend das Lösungsmittel in einem handelsüblichen Dünnschichtverdampfer bei einer Temperatur von 50°C und einem Druck von ca. 0,3 mbar ab. Der Rückstand wurde erneut filtriert und man erhielt 510 g (Ausbeute: 92,6 % d.Th.) Trihexyl-tetradecylphosphonium-1,2,4-triazolat als klare, nahezu farblose Flüssigkeit mit einer Viskosität von 570 mPas (23°C) und einem Brechungsindex $n_D^{20}$ von 1,4821. Der Restgehalt an Methanol betrug 0,1 Gew.-%.

**Beispiel 1**

1000 g (3,82 mol) 4,4'-Diisocyanatodicyclohexylmethan wurden bei 30°C unter trockenem Stickstoff und Rühren mit einer Lösung von 2 g (0,022 mol) Natrium-1,2,4-triazolat (Katalysator 1) in 25 ml Dimethylsulfoxid (DMSO) versetzt, worauf die Temperatur des Reaktionsgemisches aufgrund der freiwerdenden Reaktionswärme bis auf 39°C anstieg. Nach einer Reaktionszeit von 60 Minuten, während der die Exothermie wieder abklang, war der NCO-Gehalt der Reaktionsmischung auf 26,3 Gew.-%, entsprechend einem Oligomerisierungsgrad von 15,6 %, abgesunken. Der Katalysator wurde nun durch Zugabe von 4,6 g (0,022 mol) Dibutylphosphat deaktiviert. Die dabei entstehende Trübung wurde abfiltriert und die klare, farblose Reaktionsmischung mit Hilfe eines Dünnschichtverdampfers bei einer Temperatur von 155°C und einem Druck von 0,2 mbar von flüchtigen Bestandteilen (überschüssigem Diisocyanat und Katalysatorlösungsmittel) befreit. Man erhielt ein farbloses Uretdionpolyisocyanat mit einem Gehalt an freien NCO-Gruppen von 14,1 Gew.-%, einem Gehalt an monomerem 4,4'-Diisocyanatodicyclohexylmethan von 0,4 Gew.-%, einer Viskosität (nach DIN 53 018) von mehr als 200.000 mPas (23°C) und einer Farbzahl (APHA), bestimmt an einer 10 gew.-%igen Lösung in Methylenchlorid, von 12. Das molare Verhältnis von Uretdion- zu Isocyanuratstrukturen betrug nach $^{13}$C-NMR-Spektroskopie 98,4 : 1,6.

**Beispiel 2**

1000 g (3,82 mol) 4,4'-Diisocyanatodicyclohexylmethan wurden bei 30°C unter trockenem Stickstoff und Rühren mit einer Lösung von 2 g (0,022 mol) Natrium-1,2,3-triazolat (Katalysator 2) in 25 ml Dimethylsulfoxid (DMSO) versetzt, worauf die Temperatur des Reaktionsgemisches aufgrund der freiwerdenden Reaktionswärme bis auf 39°C anstieg. Nach einer Reaktionszeit von 60 Minuten, während der die Exothermie wieder abklang, war der NCO-Gehalt der Reaktionsmischung auf einen Wert von 26,7 Gew.-%, entsprechend einem Oligomerisierungsgrad von 14,3 %, abgesunken. Der Katalysator wurde nun durch Zugabe von 4,6 g (0,022 mol) Dibutylphosphat deaktiviert und das Reaktionsgemisch wie in Beispiel 1 beschrieben aufgearbeitet. Man erhielt ein hochviskoses farbloses Uretdionpolyisocyanat mit einem Gehalt an freien NCO-Gruppen von 14,1 Gew.-%, einem Gehalt an monomerem 4,4'-Diisocyanatodicyclohexylmethan von 0,5 Gew.-% und einer Farbzahl (APHA), bestimmt an einer 10 gew.-%igen Lösung in Methylenchlorid, von 14. Das molare Verhältnis von Uretdion- zu Isocyanuratstrukturen betrug nach $^{13}$C-NMR-Spektroskopie 99,1 : 0,9.

**Beispiel 3**

1000 g (3,82 mol) 4,4'-Diisocyanatodicyclohexylmethan wurden bei 30°C unter trockenem Stickstoff und Rühren mit einer Lösung von 3,0 g (0,021 mol) Natrium-Benztriazolat (Katalysator 3) in 40 ml Dimethylsulfoxid (DMSO) versetzt, worauf die Temperatur des Reaktionsgemisches aufgrund der freiwerdenden Reaktionswärme bis auf 37°C anstieg. Nach einer Reaktionszeit von 60 Minuten, während der die Exothermie wieder abklang, war der NCO-Gehalt der Reaktionsmischung auf einen Wert von 26,5 Gew.-%, entsprechend einem Oligomerisierungsgrad von 13,6 %, abgesunken. Der Katalysator wurde nun durch Zugabe von 4,4 g (0,021 mol) Dibutylphosphat deaktiviert und das Reaktionsgemisch wie in Beispiel 1 beschrieben aufgearbeitet. Man erhielt ein hochviskoses farbloses Uretdionpolyisocyanat mit einem Gehalt an freien NCO-Gruppen von 14,0 Gew.-%, einem Gehalt an monomerem 4,4'-Diisocyanatodicyclohexylmethan von 0,5 Gew.-% und einer Farbzahl (APHA), bestimmt an einer 10 gew.-%igen Lösung in Methylenchlorid, von 21. Das molare Verhältnis von Uretdion- zu Isocyanuratstrukturen betrug nach $^{13}$C-NMR-Spektroskopie 96,4 : 3,6.

**Beispiel 4**

1000 g (3,82 mol) 4,4'-Diisocyanatodicyclohexylmethan wurden bei 30°C unter trockenem Stickstoff und Rühren mit einer Lösung von 2,3 g (5,1 mmol) des Katalysators 4 (Tetrabutylphosphonium-1,2,4-triazolat in Methanol/Isopropanol) versetzt, worauf die Temperatur des Reaktionsgemisches aufgrund der freiwerdenden Reaktionswärme bis auf 42°C anstieg. Nach Abklingen der Exothermie wurde nach 40 Minuten mit weiteren 2,3 g (5,1 mmol) Katalysatorlösung nachkatalysiert. Nach einer Reaktionszeit von insgesamt 1 Stunde 25 Minuten betrug der NCO-Gehalt in der Reaktionsmischung 26,5 Gew.-%, entsprechend einem Oligomerisierungsgrad von 13,6 %. Der Katalysator wurde nun durch Zugabe von 2,15 g (10,2 mmol) Dibutylphosphat deaktiviert und das Reaktionsgemisch wie in Beispiel 1 beschrieben durch Dünnschichtdestillation von überschüssigem Diisocyanat befreit. Man erhielt ein hochviskoses hellgelbes Uretdionpolyisocyanat mit einem Gehalt an freien NCO-Gruppen von 14,2 Gew.-%, einem Gehalt an monomerem 4,4'-Diisocyanatodicyclo-hexylmethan von 0,4 Gew.-% und einer Farbzahl (APHA), bestimmt an einer 10 gew.-%igen Lösung in Methylenchlorid, von 17. Das molare Verhältnis von Uretdion- zu Isocyanuratstrukturen betrug nach $^{13}$C-NMR-Spektroskopie 97,2 : 2,8.

**Beispiel 5**

1000 g (3,82 mol) 4,4'-Diisocyanatodicyclohexylmethan wurden 1 Stunde am Vakuum (2 mbar) entgast, anschließend mit trockenem Stickstoff belüftet und auf 30°C erwärmt. Unter Rühren gab man 8 g (0,02 mol) des Katalysators 5 (Methyltrioctylammonium-1,2,4-triazolat) zu, wobei sich das Reaktionsgemisch aufgrund der freigesetzten Reaktionswärme auf 43°C erwärmte. Nach einer Reaktionszeit von 70 Minuten, während der die Exothermie wieder abklang, betrug der NCO-Gehalt in der Reaktionsmischung 26,6 Gew.-%, entsprechend einem Oligomerisierungsgrad von 16,2 %. Der Katalysator wurde nun durch Zugabe von 4,2 g (0,2 mol) Dibutylphosphat deaktiviert und das resultierende klare, farblose Gemisch wie in Beispiel 1 beschrieben durch Dünnschichtdestillation von überschüssigem Diisocyanat befreit. Man erhielt ein hochviskoses nahezu farbloses Uretdionpolyisocyanat mit einem Gehalt an freien NCO-Gruppen von 14,0 Gew.-%, einem Gehalt an monomerem 4,4'-Diisocyanatodicyclohexylmethan von 0,3 Gew.-% und einer Farbzahl (APHA), bestimmt an einer 10 gew.-%igen Lösung in Methylenchlorid, von 10. Das molare Verhältnis von Uretdion- zu Isocyanuratstrukturen betrug nach $^{13}$C-NMR-Spektroskopie 99,3 : 0,7.

**Beispiel 6**

1000 g (3,82 mol) 4,4'-Diisocyanatodicyclohexylmethan wurden 1 Stunde am Vakuum (2 mbar) entgast, anschließend mit trockenem Stickstoff belüftet und auf 30°C erwärmt. Anschließend gab man unter Rühren mit Hilfe einer Infusions-Laborpumpe (KDS 100, KD Scientific, Boston) 12 g (0,022 mol) des Katalysators 6 (Trihexyltetradecylphosphonium-1,2,4-triazolat) kontinuierlich über eine Reaktionszeit von 3 Stunden zu. Nach einer Nachrührzeit von 30 min betrug der NCO-Gehalt in der Reaktionsmischung 26,2 Gew.-%, entsprechend einem Oligomerisierungsgrad von 17,1 %. Der Katalysator wurde nun durch Zugabe von 4,6 g (0,022 mol) Dibutylphosphat deaktiviert und das resultierende klare, farblose Gemisch wie in Beispiel 1 beschrieben durch Dünnschichtdestillation von überschüssigem Diisocyanat befreit. Man erhielt ein hochviskoses nahezu farbloses Uretdionpolyisocyanat mit einem Gehalt an freien NCO-Gruppen von 14,2 Gew.-%, einem Gehalt an monomerem 4,4-Diisocyanatodicyclohexylmethan von 0,5 Gew.-% und einer Farbzahl (APHA), bestimmt an einer 10 gew.-%igen Lösung in Methylenchlorid, von 11. Das Produkt enthielt laut $^{13}$C-NMR-Spektroskopie ausschließlich Uretdiongruppen. Isocyanuratstrukturen waren nicht nachweisbar.

**Vergleichsbeispiel 1** (gemäß EP-A 317 744)

1000 g (3,82 mol) 4,4'-Diisocyanatodicyclohexylmethan wurden bei Raumtemperatur unter trockenem Stickstoff und Rühren mit 20 g (2 Gew.-%) 4-Dimethylaminopyridin (DMAP) als Katalysator versetzt. Nach 5 Tagen wies die fast farblose Reaktionsmischung unverändert einen NCO-Gehalt von 31,4 Gew.-% auf. Auch im IR-Spektrum fand sich kein Hinweis auf Uretdiongruppen.

**Vergleichsbeispiel 2** (gemäß EP-A 317 744)

1000 g 4,4'-Diisocyanatodicyclohexylmethan wurden wie in Vergleichsbeispiel 1 beschrieben mit 20 g (2 Gew.-%) DMAP versetzt und anschließend für 5 Tage auf 50°C erwärmt. Die schwach gelbe Reaktionsmischung wies einen unveränderten NCO-Gehalt von 31,4 Gew.-% auf. Im IR-Spektrum fand sich kein Hinweis auf Uretdiongruppen.

**Vergleichsbeispiel 3** (gemäß EP-A 317 744)

1000 g (3,82 mol) 4,4'-Diisocyanatodicyclohexylmethan wurden bei Raumtemperatur unter trockenem Stickstoff und Rühren mit 100 g (10 Gew.-%) 4-Dimethylaminopyridin (DMAP) als Katalysator versetzt. Nach 5 Tagen war im IR-Spektrum eine sehr schwach ausgeprägte Bande bei 1760 $cm^{-1}$ zu erkennen, die als Hinweis auf das Vorhandensein geringer Mengen an Uretdiongruppen gedeutet werden kann. Der NCO-Gehalt der hellgelben Reaktionsmischung war von 29,0 auf 28,6 Gew.-% gesunken, was einem Oligomerisierungsgrad von 1,4 % entspricht.

**Vergleichsbeispiel 4** (gemäß EP-A 45 995)

1000 g (3,82 mol) 4,4'-Diisocyanatodicyclohexylmethan wurden bei Raumtemperatur unter trockenem Stickstoff und Rühren mit 50 g (5 Gew.-%) Hexamethylphosphorsäuretriamid als Katalysator versetzt. Nach 5 Tagen wies die fast farblose Reaktionsmischung unverändert einen NCO-Gehalt von 31,3 Gew.-% auf. Im IR-Spektrum fand sich kein Hinweis auf Uretdiongruppen.

Die Vergleichsbeispiele zeigen, dass die literaturbekannten Katalysatoren zur hochselektiven Dimerisierung von Isocyanaten im Gegensatz zu den Katalysatoren des erfindungsgemäßen Verfahrens gegenüber sekundär gebundenen Isocyanatgruppen selbst in hohen Konzentrationen keine oder eine nur äußerst geringe, zur technischen Herstellung von Uretdionpolyisocyanaten völlig unzureichende Aktivität aufweisen.

**Beispiele 7 und 8**

Nach dem in Beispiel 6 beschriebenen Verfahren wurden-jeweils 500 g 4,4'-Diiso-cyanato-3,3'-dimethyldicyclohexylmethan (Beispiel 7) und TMXDI (Beispiel 8) bei 30°C in Gegenwart von Katalysator 6 oligomerisiert. Die Reaktionsgemische wurden jeweils mit äquimolaren Menge an Dibutylphosphat abgestoppt und anschließend durch Dünnschichtdestillation aufgearbeitet. Die nachfolgende Tabelle zeigt die jeweils eingesetzten Mengen an Katalysator und Abstopper (jeweils in Gew.-% bezogen auf die Menge an eingesetztem Ausgangsisocyanat), Reaktionszeiten sowie Kenndaten der nach Dünnschichtdestillation erhaltenen Harze.

| **Beispiel** | **7** | **8** |
|---|---|---|
| Katalysatorkonzentration [%] | 1,0 | 1,0 |
| Reaktionszeit [h] | 3,0 | 3,0 |
| Nachrührzeit [h] | 0,5 | 0,5 |
| Destillationstemperatur [°C] | 160 | 160 |
| Oligomerisierungsgrad [%] | 13,4 | 7,7 |
| NCO-Gehalt [%] | 13,3 | 15,6 |
| Monomer-Gehalt [%] | 0,6 | 0,5 |
| Farbzahl [APHA] [a)] | 29 | 43 |
| IR; Uretdionbande [$cm^{-1}$] | 1759,4 | 1764,2 |

(fortgesetzt)

| Beispiel | 7 | 8 |
|---|---|---|
| Uretdion : Isocyanurat [mol-%] [b] | 100 : 0 | 100 : 0 |
| [a] bestimmt an einer 10 gew.-%igen Lösung in Methylenchlorid<br>[b] $^{13}$C-NMR-spektroskopisch bestimmt | | |

**Beispiel 9**

100 g Cyclohexylisocyanat wurden bei Raumtemperatur unter trockenem Stickstoff mit 1 Gew.-% Katalysator 6 versetzt. Nach 24 h bei Raumtemperatur war der NCO-Gehalt von anfänglich 33,6 Gew.-% auf 29,9 Gew.-%, entsprechend einem Oligomerisierungsgrad von 10,1 %, abgesunken. Laut IR-Spektrum hatten sich ausschließlich Uretdionstrukturen (Bande bei 1760,7 $cm^{-1}$) gebildet, Isocyanuratstrukturen waren nicht nachweisbar.

**Beispiel 10**

100 g tert.-Butylisocyanat wurden bei Raumtemperatur unter trockenem Stickstoff mit 1 Gew.-% Katalysator 6 versetzt. Nach 24 h bei Raumtemperatur war der NCO-Gehalt von anfänglich 42,0 Gew.-% auf 35,7 Gew.-%, entsprechend einem Oligomerisierungsgrad von 15,0 %, abgesunken. Laut IR-Spektrum hatten sich ausschließlich Uretdionstrukturen (Bande bei 1763,5 $cm^{-1}$) gebildet, Isocyanuratstrukturen sind nicht nachweisbar.

## Patentansprüche

1. Uretdiongruppen aufweisende Verbindungen mit einen molaren Anteil an Isocyanuratstrukturen, bezogen auf die Summe an Uretdion- und Isocyanuratgruppen, von maximal 10 %, erhältlich durch Dimerisierung aliphatischer und/oder cycloaliphatischer *Mono- und/oder Diisocyanaten* mit ausschließlich sekundär und/oder tertiär gebundenen Isocyanatgruppen *in Gegenwart von salzartigen Oligomerisierungskatalysatoren, die im Anion 1,2,3- undloder 1,2,4-Triazolat-strukturen enthalten.*

2. Verbindungen gemäß Anspruch 1, die durch Dimerisierung aliphatischer und/oder cycloaliphatischer Diisocyanate mit ausschließlich sekundär und/oder tertiär gebundenen Isocyanatgruppen erhältlich sind.

3. Verbindungen gemäß Anspruch 2, die durch Dimerisierung von 4,4'-Diisocyanato-dicyclohexylmethan erhältlich sind.

4. Verfahren zur Dimerisierung von Verbindungen mit ausschließlich sekundär und/oder tertiär gebundenen Isocyanatgruppen, bei dem *aliphatische und/oder cycloaliphatische Mono- und/oder Diisocyanate,* die ausschließlich sekundär und/oder tertiär gebundene NCO-Gruppen aufweisen, in Gegenwart von salzartigen Oligomerisierungskatalysatoren, die im Anion 1,2,3- und/oder 1,2,4-Triazolatstrukturen enthalten, oligomerisiert werden.

5. Verfahren gemäß Anspruch 4, **dadurch gekennzeichnet, dass** man als Oligomerisierungskatalysatoren salzartige Verbindungen einsetzt, die im Anion Triazolatstrukturen der allgemeinen Formeln (I) und/oder (II)

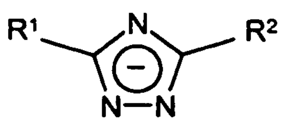

(I)

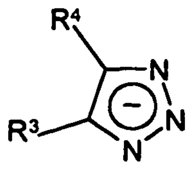

(II)

enthalten, in welchen

$R^1$, $R^2$, $R^3$ und $R^4$ für gleiche oder verschiedenen Reste stehen und jeweils ein Wasserstoffatom, ein Halogenatom aus der Reihe Fluor, Chlor oder Brom oder eine Nitrogruppe, ein gesättigter oder ungesättigter aliphatischer oder cycloaliphatischer, ein gegebenenfalls substituierter aromatischer oder araliphatischer Rest sind, der bis zu 20 Kohlenstoffatome und gegebenenfalls bis zu 3 Heteroatome aus der Reihe Sauerstoff, Schwefel, Stickstoff enthält und gegebenenfalls durch Halogenatome oder Nitrogruppen substituiert ist, und $R^3$ und $R^4$ gegebenenfalls mit den Kohlenstoffatomen des 1,2,3-Triazolatfünfringes anellierte Ringe mit 3 bis 6 Kohlenstoffatomen bilden.

**6.** Verfahren gemäß Anspruch 5, bei dem als Oligomerisierungskatalysatoren salzartige Verbindungen eingesetzt werden, die im Anion Triazolatstrukturen der allgemeinen Formel (I) enthalten, in welcher $R^1$ und $R^2$ für gleiche oder verschiedenene Reste stehen und jeweils ein Wasserstoffatom, ein Halogenatom aus der Gruppe Fluor, Chlor oder Brom oder eine Nitrogruppe, ein gesättigter aliphatischer oder cycloaliphatischer, ein gegebenenfalls substituierter aromatischer oder araliphatischer Rest sind, der bis zu 12 Kohlenstoffatome und gegebenenfalls bis zu 3 Heteroatome aus der Gruppe Sauerstoff, Schwefel, Stickstoff enthält und gegebenenfalls durch Halogenatome oder Nitrogruppen substituiert ist.

**7.** Verfahren gemäß Anspruch 5, bei dem als Oligomerisierungskatalysatoren salzartige Verbindungen eingesetzt werden, die im Anion Triazolatstrukturen der allgemeinen Formel (II) enthalten, in welcher $R^3$ und $R^4$ für gleiche oder verschiedenene Reste stehen und jeweils ein Wasserstoffatom, ein Halogenatom aus der Reihe Fluor, Chlor oder Brom oder eine Nitrogruppe, ein gesättigter oder ungesättigter aliphatischer oder cycloaliphatischer, ein gegebenenfalls substituierter aromatischer oder araliphatischer Rest sind, der bis zu 12 Kohlenstoffatome und gegebenenfalls bis zu 3 Heteroatome aus der Gruppe Sauerstoff, Schwefel, Stickstoff enthält und gegebenenfalls durch Halogenatome oder Nitrogruppen substituiert ist, oder gemeinsam mit den Kohlenstoffatomen des 1,2,3-Triazolatfünfringes anellierte Ringe mit 3 bis 6 Kohlenstoffatomen bilden.

**8.** Verfahren gemäß Anspruch 5, bei dem als Oligomerisierungskatalysatoren Salze des 1,2,4-Triazols, des 1,2,3-Triazols und/oder des 1,2,3-Benztriazols eingesetzt werden.

**9.** Verfahren gemäß Anspruch 4, **dadurch gekennzeichnet, dass** man Oligomerisierungskatalysatoren einsetzt, die als Kationen Alkaliionen oder einwertige Ammonium- oder Phosphoniumkationen, der allgemeinen Formel (III)

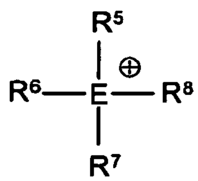

(III)

enthalten, in welcher

E für Stickstoff oder Phosphor steht und
$R^5$, $R^6$, $R^7$ und $R^8$ für gleiche oder verschiedenen Reste stehen und jeweils einen gesättigten aliphatischen oder cycloaliphatischen, einen gegebenenfalls substituierten aromatischen oder araliphatischen Rest mit bis zu 18 Kohlenstoffatome bedeuten.

**10.** Verwendung der Uretdiongruppen aufweisenden Verbindungen gemäß Anspruch 1 als Ausgangskomponenten bei der Herstellung von Polyurethankunststoffen.

**11.** Verwendung der Uretdiongruppen aufweisenden Polyisocyanate gemäß Anspruch 1 als Ausgangskomponenten bei der Herstellung von Uretdionpulverlackvernetzern.

## Claims

**1.** Compounds containing uretdione groups, having a molar fraction of isocyanurate structures, based on the sum of uretdione groups and isocyanurate groups, of not more than 10%, obtainable by dimerizing aliphatic and/or cycloaliphatic mono-and/or diisocyanates containing exclusively secondary and/or tertiary isocyanate groups in the presence of saltlike oligomerization catalysts which contain 1,2,3- and/or 1,2,4-triazolate structures in the anion.

**2.** Compounds according to Claim 1, obtainable by dimerizing aliphatic and/or cycloaliphatic diisocyanates containing exclusively secondary and/or tertiary isocyanate groups.

**3.** Compounds according to Claim 2, obtainable by dimerizing 4,4'-diisocyanatodicyclohexylmethane.

**4.** Process for dimerizing compounds containing exclusively secondary and/or tertiary isocyanate groups, in which aliphatic and/or cycloaliphatic mono- and/or diisocyanates, which comprise exclusively secondary and/or tertiary NCO groups are oligomerized in the presence of saltlike oligomerization catalysts which contain 1,2,3-and/or 1,2,4-triazolate structures in the anion.

**5.** Process according to Claim 4, **characterized in that** as oligomerization catalyst saltlike compounds are used containing in the anion triazolate structures of the general formulae (I) and/or (II)

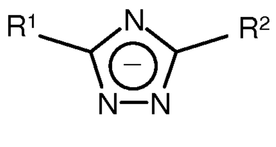

(I)

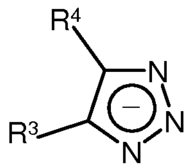

(II)

in which

$R^1$, $R^2$, $R^3$ and $R^4$ are identical or different radicals and are each a hydrogen atom, a halogen atom from the fluorine, chlorine or bromine series or a nitro group, are a saturated or unsaturated aliphatic or cycloaliphatic radical or an optionally substituted aromatic or araliphatic radical which contains up to 20 carbon atoms and optionally up to 3 heteroatoms from the oxygen, sulphur and nitrogen series and is optionally substituted by halogen atoms or nitro groups, and optionally $R^3$ and $R^4$ with the carbon atoms of the 1,2,3-triazolate five-membered ring form fused rings having 3 to 6 carbon atoms.

**6.** Process according to Claim 5, in which as oligomerization catalysts saltlike compounds are used containing in the anion triazolate structures of the general formula (I) in which $R^1$ and $R^2$ are identical or different radicals and are each a hydrogen atom, a halogen atom from the fluorine, chlorine or bromine group or a nitro group, are a saturated aliphatic or cycloaliphatic radical or an optionally substituted aromatic or araliphatic radical which contains up to 12 carbon atoms and optionally up to 3 heteroatoms from the oxygen, sulphur and nitrogen group and is optionally substituted by halogen atoms or nitro groups.

**7.** Process according to Claim 5, in which as oligomerization catalysts saltlike compounds are used containing in the anion triazolate structures of the general formula (II) in which $R^3$ and $R^4$ are identical or different radicals and are each a hydrogen atom, a halogen atom from the fluorine, chlorine or bromine series or a nitro group, are a saturated or unsaturated aliphatic or cycloaliphatic radical or an optionally substituted aromatic or araliphatic radical which contains up to 12 carbon atoms and optionally up to 3 heteroatoms from the oxygen, sulphur and nitrogen group and is optionally substituted by halogen atoms or nitro groups and together with the carbon atoms of the 1,2,3-triazolate five-membered ring form fused rings having 3 to 6 carbon atoms.

**8.** Process according to Claim 5, in which as oligomerization catalysts use is made of salts of 1,2,4-triazole, of 1,2,3-triazole and/or of 1,2,3-benzotriazole.

**9.** Process according to Claim 4, **characterized in that** oligomerization catalysts are used containing as cations alkali metal ions or monovalent ammonium or phosphonium cations, of the general formula (III)

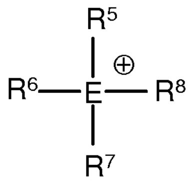

(III)

in which

E is nitrogen or phosphorus and
$R^5$, $R^6$, $R^7$ and $R^8$ are identical or different radicals and are each a saturated aliphatic or cycloaliphatic radical or an optionally substituted aromatic or araliphatic radical having up to 18 carbon atoms.

**10.** Use of the compounds containing uretdione groups according to Claim 1 as starting components for the preparation of polyurethane polymers.

**11.** Use of the polyisocyanates containing uretdione groups according to Claim 1 as starting components for the preparation of uretdione powder coating crosslinkers.

## Revendications

**1.** Composés présentant des groupes uretdione, dotés d'une proportion molaire de structures isocyanurate, par rapport à la somme des groupes uretdione et isocyanurate, d'au maximum 10%, pouvant être obtenus par dimérisation de monoisocyanates et/ou de diisocyanates aliphatiques et/ou cycloaliphatiques présentant des groupes isocyanate liés exclusivement de manière secondaire et/ou tertiaire en présence de catalyseurs d'oligomérisation contenant un sel, qui contiennent des structures 1,2,3-triazolate et/ou 1,2,4-triazolate dans l'anion.

**2.** Composés selon la revendication 1 qui peuvent être obtenus par dimérisation de diisocyanates aliphatiques et/ou cycloaliphatiques présentant des groupes isocyanate liés exclusivement de manière secondaire et/ou tertiaire.

**3.** Composés selon la revendication 2, qui peuvent être obtenus par dimérisation de 4,4'-diisocyanatodicyclohexylméthane.

**4.** Procédé pour la dimérisation de composés présentant des groupes isocyanate liés exclusivement de manière secondaire et/ou tertiaire, dans lequel on oligomérise des monoisocyanates et/ou des diisocyanates aliphatiques et/ou cycloaliphatiques, qui présentent des groupes NCO liés exclusivement de manière secondaire et/ou tertiaire, en présence de catalyseurs d'oligomérisation contenant un sel, qui contiennent des structures 1,2,3-triazolate et/ou 1,2,4-triazolate dans l'anion.

**5.** Procédé selon la revendication 4, **caractérisé en ce qu'**on utilise, comme catalyseurs d'oligomérisation, des composés contenant du sel qui contiennent dans l'anion des structures triazolate des formules générales (I) et/ou (II)

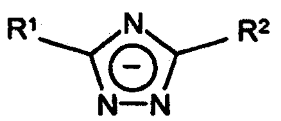

(I)

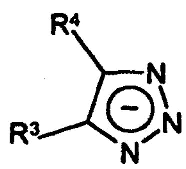

(II)

dans lesquelles

$R^1$, $R^2$, $R^3$ et $R^4$ représentent des radicaux identiques ou différents et représentent à chaque fois un atome d'hydrogène, un atome d'halogène de la série fluor, chlore ou brome ou un groupe nitro, un radical saturé ou

insaturé, aliphatique ou cycloaliphatique, un radical aromatique ou araliphatique le cas échéant substitué, qui contient jusqu'à 20 atomes de carbone et le cas échéant jusqu'à 3 hétéroatomes de la série oxygène, soufre, azote et qui est le cas échéant substitué par des atomes d'halogène ou des groupes nitro et $R^3$ et $R^4$ forment le cas échéant, avec les atomes de carbone du pentagone 1,2,3-triazolate, des cycles annelés comprenant 3 à 6 atomes de carbone.

**6.** Procédé selon la revendication 5, dans lequel on utilise, comme catalyseurs d'oligomérisation, des composés contenant un sel, qui contiennent dans l'anion des structures triazolate de formule générale (I), dans laquelle $R^1$ et $R^2$ représentent des radicaux identiques ou différents et représentent à chaque fois un atome d'hydrogène, un atome d'halogène de la série fluor, chlore ou brome ou un groupe nitro, un radical saturé ou insaturé, aliphatique ou cycloaliphatique, un radical aromatique ou araliphatique le cas échéant substitué, qui contient jusqu'à 12 atomes de carbone et le cas échéant jusqu'à 3 hétéroatomes du groupe oxygène, soufre, azote et qui est le cas échéant substitué par des atomes d'halogène ou des groupes nitro.

**7.** Procédé selon la revendication 5, dans lequel on utilise, comme catalyseurs d'oligomérisation, des composés contenant un sel, qui contiennent dans l'anion des structures triazolate de formule générale (II), dans laquelle $R^3$ et $R^4$ représentent des radicaux identiques ou différents et représentent à chaque fois un atome d'hydrogène, un atome d'halogène de la série fluor, chlore ou brome ou un groupe nitro, un radical saturé ou insaturé, aliphatique ou cycloaliphatique, un radical aromatique ou araliphatique le cas échéant substitué, qui contient jusqu'à 12 atomes de carbone et le cas échéant jusqu'à 3 hétéroatomes du groupe oxygène, soufre, azote et qui est le cas échéant substitué par des atomes d'halogène ou des groupes nitro ou forment ensemble avec les atomes de carbone du pentagone 1,2,3-triazolate des cycles annelés le 3 à 6 atomes de carbone.

**8.** Procédé selon la revendication 5, dans lequel on utilise, comme catalyseurs d'oligomérisation, des sels du 1,2,4-triazole, du 1,2,3-triazole et/ou du 1,2,3-benzotriazole.

**9.** Procédé selon la revendication 4, **caractérisé en ce qu'**on utilise des catalyseurs d'oligomérisation qui contiennent, comme cation, des ions de métal alcalin ou des cations monovalents d'ammonium ou de phosphonium, de formule générale (III)

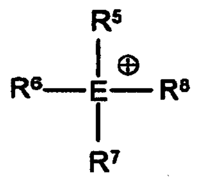

(III)

dans laquelle

E représente azote ou phosphore et
$R^5$, $R^6$, $R^7$ et $R^8$ représentent des radicaux identiques ou différents et signifient à chaque fois un radical saturé aliphatique ou cycloaliphatique, un radical aromatique ou araliphatique le cas échéant substitué comprenant jusqu'à 18 atomes de carbone.

**10.** Utilisation des composés présentant des groupes uretdione selon la revendication 1 comme composants de départ lors de la préparation de matériaux synthétiques à base de polyuréthane.

**11.** Utilisation des polyisocyanates présentant des groupes uretdione selon la revendication 1 comme composants de départ lors de la préparation de réticulants de laque en poudre à base d'uretdione.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 45996 A
- EP 639598 A
- EP 669353 A
- EP 45995 A
- EP 317744 A
- EP 735027 A
- EP 896973 A
- EP 178520 A
- DE 3420114 A


### In der Beschreibung aufgeführte Nicht-Patentliteratur


- *J. Prakt. Chem.,* 1994, vol. 336, 185-200
- *Justus Liebigs Annalen der Chemie,* 1949, vol. 562, 75-136